# EUROPEAN PATENT SPECIFICATION

(11) **EP 1 615 559 B1**
(45) Date of publication and mention of the grant of the patent: **22.08.2012**
(21) Application number: 04727653.0
(22) Date of filing: 15.04.2004
(51) Int. Cl.: A61B 5/06, A61K 49/18

(54) **METHOD TO DETERMINE THE SPATIAL DISTRIBUTION OF MAGNETIC PARTICLES AND MAGNETIC PARTICLE ADMINISTERING COMPOSITIONS**
VERFAHREN ZUR ERMITTLUNG DER RÄUMLICHEN VERTEILUNG MAGNETISCHER PARTIKEL SOWIE ZUSAMMENSETZUNG ZU DEREN GABE
PROCEDE DE DETERMINATION DE LA REPARTITION SPATIALE DE PARTICULES MAGNETIQUES ET COMPOSITIONS DESTINEES A L'ADMINISTRATION DE PARTICULES MAGNETIQUES

(30) Priority: 15.04.2003 EP 03101017
(43) Date of publication of application: 18.01.2006
(73) Proprietor: Philips Intellectual Property & Standards GmbH, 20099 Hamburg (DE); Koninklijke Philips Electronics N.V., 5621 BA Eindhoven (NL)
(72) Inventor: GLEICH, Bernhard, Philips In. Pro. & Stan. GmbH, 52066 Aachen (DE); WEIZENECKER, Jürgen, Philips In. Pro. & Stan. GmbH, 52066 Aachen (DE)
(74) Representative: Volmer, Georg
(86) International application number: PCT/IB2004/050444
(87) International publication number: WO 2004/091394

(56) References cited:
- WO-A-85/02772
- US-A- 4 827 945
- US-A- 5 792 445
- SHEN L ET AL: "Aqueous magnetic fluids stabilized by surfactant bilayers" JOURNAL OF MAGNETISM AND MAGNETIC MATERIALS, ELSEVIER SCIENCE PUBLISHERS, AMSTERDAM, NL, vol. 194, no. 1-3, April 1999 (1999-04), pages 37-44, XP004166644 ISSN: 0304-8853 cited in the application

## Description

The present invention relates to a method to determine the spatial distribution of magnetic particles in an examination area of an object of examination and to magnetic particle administering compositions. Imaging methods using magnetic particles as contrast agents are known. For example, in the MRI method (Magnetic Resonance Imaging), iron oxide is used as the contrast agent to influence relaxation times.

DE 37 51 918 T2 describes a method to obtain an in-vivo image of an animal or human organ or tissue with the aid of nuclear magnetic resonance technology, in which an image-improving dose of nuclear magnetic resonance contrast agent, in the form of a superparamagnetic fluid, prepared in a specific manner, is used. The magnetic contrast agent influences the magnetic properties of the tissue under examination so that the irradiated protons evidence improved relaxation behaviour. Superparamagnetic and ferromagnetic substances make the magnetic resonance image appear darker due to the reduction of T₂. However, suitable contrast agents for nuclear spin tomography require extremely stable solutions to effectively increase the sensitivity of magnetic resonance measurement. The stability of suitable aqueous fluids of superparamagnetic iron oxides is frequently limited considerably by clumping due to magnetic forces of attraction between particles. DE 37 51 918 T2 proposes a four-stage method for the production of a stable superparamagnetic fluid comprising bivalent and trivalent metal salts. This method is very time and cost intensive and is therefore not necessarily suitable for standard examinations. Although the magnetic particles obtained with this method can be used to increase anatomical and physiological contrast, they are not generally suited to make the acquisition of parameters such as temperature and pH values using MRI technology more accurate and faster. In addition, nuclear spin tomography requires the use of very strong magnetic fields with high homogeneity. In general, superconducting coils are used together with liquid helium cooling. The nuclear spin tomography method is therefore always linked with a high equipment outlay.

According to Chupp and Swanson, "Medical Imaging with Laser Polarized Noble Gases", Advances in Atomic, Molecular and Optical Physics, 45, 41 (2001), signals generated with magnetic resonance (MR) and nuclear magnetic resonance (NMR) methods can be amplified by several orders of magnitude when laser-irradiated polarized noble gases, particularly ³He and ¹²⁹Xe, are introduced into the area of examination. ³He is particularly suitable for lung imaging, while ¹²⁹Xe is used, e.g. for the imaging of organs. The production of these noble gases polarized by laser radiation requires extensive equipment. In addition, it is difficult to produce large quantities of these polarized noble gases. Special measures must also be undertaken to ensure that the polarized noble gases are not contaminated by oxygen, even trace amounts, as this will essentially negate the signal amplifying effect.

Post published document EP 1 304 542 A discloses a method that makes use of a novel imaging technique called "Magnetic Particle Imaging" (MPI). This method involves generating a magnetic field to produce in an examination region a first sub-region of low magnetic field strength, and a second sub-region of high magnetic field strength. The spatial position of the two sub-regions is varied so that the magnetization of the particles varies locally. Signals are detected that depend on the magnetization influenced by this variation. The signals are evaluated to obtain information relating to the spatial distribution of the magnetic particles in the examination region.

It is an object of the present invention to provide an imaging method using magnetic phenomena with which data, particularly data suitable for imaging, can be generated in a reproducible and precise manner with simple and cost-effective equipment from the area of examination of an object of examination.

This object is achieved by providing a method to determine the spatial distribution of magnetic particles which have been introduced in an examination area of an object of examination, said method including the following steps:
a) Generation of a magnetic field with a spatial distribution of the magnetic field strength such that the examination area consists of a first sub-area with lower magnetic field strength and a second sub-area with a higher magnetic field strength,
b) Change of the particularly relative spatial position of the two sub-areas in the area of examination or change of the magnetic field strength in the first sub-area so that the magnetization of the magnetic particles which have been introduced in the area of examination in a suspension, aerosol, in the form of a powder, especially diluted, with a casing or, especially, a thin coating, present in at least one capsule, or coupled to cells, particularly white or red blood corpuscles, immune cells, tumor cells or stem cells, or to ingredients, medication, antibodies, transplants or living organisms, or in the form of a, especially liquid, precursor form, changes locally,
c) Acquisition of signals that depend on the magnetization in the area of examination influenced by this change, and
d) Evaluation of said signals to obtain information about the change in spatial distribution and/or the movement of the magnetic particles in the area of examination. It is envisaged that an embodiment may include at least a repetition of steps b) to d).

Magnetic particles in a suspension are suitable for instance for intravenous injections, while magnetic particles in an aerosol are preferred for lung imaging or imaging of the breathing system. Magnetic particles in the form of a powder, especially a diluted powder, can be introduced either directly, or as a suspension or aerosol, into the area of examination. The encasing, or more particularly thin coating, of magnetic particles can prevent clumping or agglomeration of the particles during storage or introduction to, or while in, the area of examination. Preferably, temporary coatings or casings are used. Temporary coatings or casings can be made, e.g. of polysaccharides such as dextrane or viscous gels which, depending on the conditions in the area of examination, can partially or fully dissolve.

In a further embodiment, the magnetic particles are contained in a capsule or a coating and are brought in this form to the area of examination. Suitable capsule or coating materials are those that decompose and release the particles under certain conditions in the area of examination, e.g. at a specific pH value or temperature. In addition, a capsule material can be selected that dissolves when exposed to ultrasound or light.

A preferred embodiment of the method according to the invention is characterized in that the material used for encasing or coating the magnetic particles used can be degraded or dissolved thermally, chemically, biochemically, by means of electromagnetic radiation or ultrasound and/or mechanically. It may then be provided that the material for the encasing or coating comprises polysaccharides, starches, particularly dextrin or cyclodextrin, waxes, oils, fats, glycerin, gels or plastics, particularly thermoplastic polymers or their blends. Furthermore it may be provided that the magnetic particles have at least a partially coating or casing of at least one protein, polypeptide, antibody and/or organosilanes.

The coating of magnetic particles with biologically degradable materials, e.g. with dextranes and proteins, is described in DE 37 51 918 T2. Particle coatings with organic polymers are also described by Shen et al., J. Magn. Magn. Mater. 1999, 194, pages 37 ff., and by Del Gratta et al., Phys. Med. Biol. 1995, 40, pages 671 ff. The coating of magnetic particles can also be found in EP 186 616 A1.

Another aspect of the present invention also provides the introduction of magnetic particles coupled to cells, especially e.g. white or red blood corpuscles, immune cells, tumor cells and/or stem cells into the area of examination. The coupling of magnetic particles to white blood corpuscles or immune cells enables the precise localization and imaging of sources of inflammation. The coupling of magnetic particles, especially magnetic nano-particles, to blood cells is described, e.g. by Groß et al., in "Jahrestagung der Deutschen Pharmazeutischen Gesellschaft", Münster, 2000. By coupling these particles to tumor cells or antibodies, it is possible to achieve very precise examination of tumour growth or propagation of tumors or metastasis formation. Administering forms are preferred where magnetic particles, coupled to medication, transplants or living organisms, are brought into and/or are present in the area of examination. In this manner it is possible to observe where the object coupled to the magnetic particles is transported to and by which route and speed. Living organisms marked with magnetic particles can comprise, e.g. bacteria or even insects, such as lice.

In a further embodiment of the present invention a precursor of magnetic particles is introduced to the area of examination. This has the advantage that not just magnetic particles present in solid form can be introduced to the area of examination, but that this introduction can take place with suitable solutions.

In particular, there is provided a method for administering a magnetic particle composition to an examination object, wherein the examination object is contacted with a first solution comprising ferrous and ferric ions and, before or after that, contacted with a second solution comprising a base to precipitate the magnetic particles in the examination area. A preferred embodiment provides that a precursor comprises a first aqueous solution containing FeCl₂ and FeCl₃, and a second aqueous solution containing NaOH, is introduced to the area of examination where the first and second solutions come into contact and form magnetic particles.

A magnetic particle formation kit for use in the above-described method can be provided, wherein the kit comprises a first container comprising a first aqueous solution of ferrous and ferric ions and a second container comprising a second basic solution. The composition is chosen such that under the circumstances prevailing in the examination area magnetic particles are formed having good properties for magnetic particle imaging, in particular having eight step change in the magnetisation curve as specified below.

With this administering method, objects can be examined where magnetic particles cannot be introduced in any other manner. For example, this applies to plastic materials or ceramic materials, which could be examined with the method according to the invention for, e.g. hairline cracks. The liquid precursor described above can penetrate such cracks and then form magnetic particles inside the object of examination. The described administering method is also particularly suitable for the examination or measurement of boreholes or other hollow spaces for visualising and studying porosity or hair cracks in a material.

An administering composition for administering of a magnetic particle composition into an examination area can be provided, comprising administering particles containing one or more magnetic particles in a first coating material, which first coating material is easily removed in conditions prevailing in the examination area. The advantage of this administering composition is that the magnetic particles are prevented from clumping or agglomerating. The administering composition can be stored in a dry particle powder form, is stable and has a long shelf life. After administering the particles into the examination area, the coating is removed, for example by dissolving or by degradation, thus removing the restriction for the magnetic particle to move into the examination area and to respond to the external magnetic fields. Preferably, the first coating material is at least partly removed in less than about 20 seconds after administering into the examination area. Suitable first coating materials are polysaccharide or starch.

The administering particles comprise only one magnetic particle coated with the first coating material and the diameter of the administering particle is at least 5 times, preferably at least 10 times the diameter of the magnetic particle. The advantage of this is that the magnetic particles after being released from the administering particle do not easily re-agglomerate in the examination area.

The administering particles may comprise two or more magnetic particles and the two or more magnetic particles are at an average distance of at least 5 times, preferably at least 10 times the average diameter of the magnetic particles. If, the magnetic particles after administering in the examination area are likely to move away from each other, for example by transport in a flowing medium (bloodstream) or by dilution, a relatively small distance can be chosen, whereas in more static media a distance of at least 10 times the average diameter is preferred.

The magnetic particles in the administering particles can be individually coated with a second coating material different from the first coating material. The individually coated magnetic particles are embedded in the first coating material. The function of the second coating material preferably differs from the function of the first coating material for example to improve biocompatibility.

Further, it is preferred that the administering particles in the administering composition comprise a further outer coating of a material different from the first or second coating material. For example, the administering particles comprise an easy dissolvable first coating material and a more water resistant outer coating material to improve storage stability of the administering composition.

The administering composition can be administered into the examination area directly, directly after dispersing the administering composition in a suitable liquid medium or the administering composition can be pre-dispersed in a liquid to at least partly remove the coating before administering into the examination area. In this method it is preferred that the liquid comprising the at least partly dissolved administering composition is added to the examination area before substantial agglomeration and clumping of the magnetic particles as occurred, typically within about 20 seconds after removal of the coating.

In general, areas of examination comprise any areas where the described administering methods can be introduced. It is also provided that the area of examination is present in the lungs, sinuses or other parts of the breathing system, in the digestive system, inner ears, bladder, vagina, mammary glands, circulation system, particularly the heart, liver, spleen, lymph system, bone marrow and more particularly in inflamed organs and/or tumors.

An alternative embodiment of the present method according to the invention provides that the area of examination may comprise boreholes or materials made of plastic or ceramic.

In addition, the object of examination in which the area of examination is present can comprise a polymer material, especially a thermoplastic polymer, or polymer blend, a polymer melt, a micro-organism, a plant component, an organism or a portion of an organism.

Basically, the method according to the invention can be used to examine any object, regardless of composition, consistency, form or size. For instance, fluid, viscous and solid objects of examination can be analyzed with the method according to the invention.

For administering magnetic particles to gas filled spaces preferably, aerosol particles are used. The aerosol administering composition is administered for example in the respiratory system or spaces that are difficult to access for investigation, for example cavities or porosity in materials, piping vessels earth formations etc. An aerosol administering composition for administering of a magnetic particle composition into an examination area can also be provided, wherein the particles have a diameter below 100 m, preferably below 10µm, and wherein the particles are from a hard magnetic material. Preferably, the magnetisation reversal by Neel rotation of the magnetic particles does not take place below 10 mT. With the above aerosol administering composition particles are easily magnetised whilst they are in the aerosol state and are not easily magnetised when absorbed or in contact with the walls surrounding the cavity. The contrast in the magnetic particle imaging technique is obtained from the difference between the easy magnetisable aerosol particles and the particles that are hindered in geometric rotation and not easily reverse magnetisation in an external magnetic field.

An administering composition for investigating small vessels may comprise particles having a size at least 8, preferably at least 10 micrometer, which particles comprise a magnetic particle and optionally a coating material, which magnetic particle and optional coating material slowly degrade in the vessels. As the size of these particles is approximately the size of the small blood vessels, the particles get stuck and accumulate in the small blood vessels. This provides information on the presence and perfusion of small blood vessels in certain tissues, for example, tumour tissue and lung perfusion. Preferably, the coated particle has a size less than 150µm more preferred less than 90µm. Preferably, the magnetic particle is a needle shaped multi-domain particle, composed of aligned smaller particles wherein the magnetic vectors of the smaller particles are largely oriented along the needle axis and which needle shaped particle degrades to the individual small particles in the vessels. The needles are preferably coated, e.g. with albumin, to form a more or less spherical particle. Preferably, the time to degrade the particles is at least 10 minutes.

The method according to the invention uses the circumstance that magnetic particles that are not saturated can be influenced by an external magnetic field and their reaction to the external magnetic field can be detected. Conclusions about the environment in which the magnetic particles are present can be made in this manner. A reaction to or interaction with an applied external field, i.e. magnetic reversal, can be easily generated, with a particularly anisotropic magnetic particle, when this particle is not hindered from aligning itself in the direction of the external magnetic field lines by, e.g. mechanical, influences. In how far the behaviour of the magnetic particle in the area of examination is dependent on its direct environment can, for example, be precisely detected when this magnetic particle changes state or gains/loses movement.

The method according to the invention therefore makes significant use of an arrangement as described in the German patent number 10151 778. For preferred embodiments of this arrangement reference is made to the above patent application.

The arrangement used in the invention generates a spatially inhomogeneous magnetic field in the area of examination. In the first sub-area, the magnetic field is so weak that the magnetization of the particle deviates more or less strongly from the external field and is therefore not saturated. This first sub-area is preferentially a spatially coherent area; it can however also be a punctiform area, but also a line or a plane. In the second sub-area (i.e. the rest of the area of examination lying outside the first area), the magnetic field is sufficiently strong to hold the particle in a state of saturation. Magnetization is saturated when the magnetization has aligned almost all particles in approximately the direction of the external magnetic field so that with an increase in magnetic field, the magnetization in that area increases considerably less than in the first sub-area with a similar increase in magnetic field.

By changing the position of the two sub-areas within the area of examination, the (total) magnetization in the area of examination changes. If, therefore, the magnetization in the area of examination or the physical parameters influenced by this are measured, information can be derived about the spatial distribution of the magnetic particles in the area of examination.

To change the spatial position of both sub-areas in the area of examination or to change the magnetic field strength in the first sub-area, an e.g. magnetic field that is localized and/or changes over time can be generated. It is also provided that the signals induced in at least one coil by the change over time of the magnetization in the area of examination are acquired and evaluated to obtain information about the spatial distribution of magnetic particles in the area of examination. The biggest possible signals are achieved by changing the spatial position of both sub-areas as rapidly as possible. A coil, with which a magnetic field can be generated in the area of examination, can be used to acquire the signals. Preferably, at least one separate coil is used.

When changing the spatial position of the sub-area using a magnetic field changing over time, this can induce a similarly periodic signal in a coil. The acquisition of this signal may however be difficult as the signals generated in the area of examination and the magnetic field changing over time are simultaneously effective: it is therefore not possible to differentiate between the signals induced by the magnetic field and the signals induced by the change in magnetization in the area of examination. This can however be avoided in that a magnetic field changing over time acts on a first frequency band on the area of examination and that a second frequency band, which contains higher frequency components than the first frequency band, in the signal received from the coil is evaluated to obtain information about the spatial distribution of the magnetic particles. This exploits the fact that the frequency components of the second frequency band can only be created by a change in the magnetization in the area of examination due to the non-linearity of the magnetization characteristic curve. When the magnetic field changing over time has a sinusoidal periodic behaviour, the first frequency band consists only of a single frequency component - the sinusoidal fundamental oscillation. In contrast, the second frequency band contains, in addition to this fundamental oscillation, higher harmonics (so-called harmonic waves) of the sinusoidal fundamental oscillation, which can be used for evaluation.

A preferred arrangement for the method in the present invention is characterized in that the means for generating the magnetic field includes a gradient coil arrangement for generating a magnetic gradient field, which reverses its direction in the first sub-area of the area of examination and evidences a zero passage. This magnetic field is - when the gradient coil arrangement e.g. comprises two identical windings carrying opposing flows located on either side of the area of examination (Maxwell coil) - zero at a point on the winding axis and increases almost linearly on both sides of this point with opposite polarities. It is only with these particles located in the area around this field zero point where magnetization is not saturated. For particles outside this area, the magnetization is in a state of saturation.

Therefore an arrangement can be provided with means to generate a magnetic field changing over time and superimposed on the magnetic gradient field for the purpose of moving both sub-areas in the area of examination. The area generated by the gradient coil. arrangement is therefore moved around the field zero point, i.e. the first sub-area, within the area of examination by the magnetic field changing over time. With appropriate changes over time and orientation of this magnetic field it is possible to move the field zero point throughout the entire area of examination.

The magnetization change resulting from the movement of the field zero point can be detected by an appropriate coil arrangement. The coil used to detect the signals generated in the area of examination can be a coil that is already used to generate the magnetic field in the area of examination. There are, however, advantages to using a separate coil for reception as this can be decoupled from the coil arrangement producing a magnetic field that changes over time. In addition, an improved signal/noise ratio can be achieved with a coil - and more so with several coils.

The amplitude of the signals induced in the coil arrangement increases proportionally the faster the position of the field zero point changes in the area of examination, i.e. the faster the magnetic field changing over time superimposed on the magnetic gradient field changes. It is however technically difficult to generate a magnetic field changing over time with sufficient amplitude to move the field zero point at the point of the area of examination or with sufficiently large change speed to generate signals with sufficient amplitude. Particularly suitable arrangements for this purpose comprise means to generate a first and at least a second magnetic field superimposed on the magnetic gradient field, whereby the first magnetic field moves slowly with high amplitude and the second magnetic field moves fast with low amplitude. This generates - preferably by two coil arrangements - two magnetic fields with different speeds and different amplitudes. Another advantage is that the field changes can be so fast (e.g. > 20 kHz) that they lie above the human limit of audibility. It can also be provided that both magnetic fields in the area of examination are generally aligned vertically to one another. This enables the movement of the field-free point within a two-dimensional area. This can be expanded to a three-dimensional area by another magnetic field comprising a component aligned vertically to the two magnetic fields. Another advantage is inherent in an arrangement with a filter downstream of a coil arrangement, which suppresses the signal components in a first frequency band in the signal induced, by the coil arrangement and allows the signal components in a second frequency band, which contains higher frequency components than the first frequency components, to pass. This exploits the fact that the magnetization characteristic curve is non-linear in the area where the magnetization transitions from the non-saturated to the saturated state. This non-linearity has the effect that, e.g. a sinusoidal magnetic field over time with the frequency f generates, in the area of non-linearity, an induction changing over time with the frequency f (fundamental oscillation) and integer multiples of the frequency f (harmonic waves or higher harmonics). The evaluation of the higher harmonics has the advantage that the fundamental oscillation of the magnetic field used to move the field-free point does not have any influence on the evaluation.

It has been found to be effective for some applications, for example for viscosity or flow measurements, that at least a portion of the magnetic particles has anisotropic properties

It can be provided that the magnetic particle is a mono-domain particle whose magnetic reversal is implemented mainly through Brownian rotation or Neel rotation.

In another suitable embodiment of the method according to the invention, the magnetic particle may be represented by a hard or soft magnetic multi-domain particle.

In another embodiment, the magnetic particles comprise hard magnetic materials. It may then be that the hard magnetic materials comprise Al-Ni, Al-Ni-Co and Fe-Co-V alloys as well as barium ferrite (BaO 6xFe₂O₃).

According to another aspect of the method, it is proposed that the material used for encasing or coating can be degraded or dissolved thermally, chemically, biochemically, by means of electromagnetic radiation or ultrasound and/or mechanically.

A preferred embodiment provides that the magnetic particles comprise superparamagnetic particles or ferromagnetic particles, particularly in the form of flakes or needles.

According to the present invention, it is provided that the magnetic particles become saturated when an external magnetic field is applied, especially one with a strength of circa 100 mT or less. Of course, larger saturation field strengths are also suitable for the method according to the invention.

Suitable magnetic field strengths for many applications are circa 10 mT or less. This strength would already be sufficient for many tissue or organ examinations. But it is also possible to achieve good measurement results with field strengths in the area of 1 mT or less, or circa 0.1 mT or less. For example, concentration data, temperature, pressure or pH values can be determined with high accuracy and resolution with magnetic fields of circa 10 mT or less, circa 1 mT or less and circa 0.1 mT or less.

In the sense of the present invention, an external magnetic field where the magnetic particles become or are saturated means a magnetic field where circa half the saturation magnetization is achieved.

Suitable magnetic particles here are those that can reach saturation with a sufficiently small magnetic field. A necessary requirement for this is that the magnetic particles have a minimum size or a minimum dipole moment. The term magnetic particle in the sense of the present invention also comprises particles that can be magnetized.

Suitable magnetic particles favourably have dimensions that are small compared to the size of the voxel whose magnetization is to be determined by the method according to the invention. In addition, the magnetization of the particles should preferably reach saturation at the lowest possible field strengths of the magnetic field. The lower the field strength required for this is, the higher the spatial resolution capacity or the weaker the (external) magnetic field being generated in the area of examination can be. In addition, the magnetic particles must have the highest possible dipole moment or a high saturation induction so that the change in magnetization produces the largest possible output signals. It is also important for the particles not to be toxic if the method is to be used for medical examinations.

A preferred form of the present method according to the invention proposes that the magnetic particle is a mono-domain particle that can be reverse magnetized by Neel rotation and/or that the reverse magnetization is caused by Brownian rotation.

Suitable magnetic mono-domain particles are preferably dimensioned so that only a single magnetic domain (the mono-domain) can be formed in them or Weiß areas are not present. Suitable particle sizes in a specially preferred embodiment of the present invention lay in the range between 20 nm to ca. 800 nm, where the upper limit is also dependent on the material used. Preferably, magnetite (Fe₃O₄), maghemite (γ-Fe₂O₃) and/or non-stoichiometric magnetic iron oxides are used as mono-domain particles.

In general it is advantageous, especially when a rapid reverse magnetization based on Neel rotation is required, for the mono-domain particles to evidence a low effective anisotropy. Effective anisotropy means the anisotropy resulting from the form anisotropy and the average crystal anisotropy. In the above-mentioned case a change in magnetization direction does not require the particle to be turned. Alternatively, mono-domain particles with high effective anisotropy can be used when it is desired that the reverse magnetization, when applying an external magnetic field, is implemented by Brownian or geometric rotation. Above all, particles whose reverse magnetization is based on Neel rotation and on Brownian rotation are particularly suitable for viscosity measurements.

Another embodiment of the present method according to the invention proposes that the magnetic particle may be represented by a hard or soft magnetic multi-domain particle. These multi-domain particles are usually larger magnetic particles in which a number of magnetic domains can be formed. Such multi-domain particles suitably have a low saturation induction.

Hard magnetic multi-domain particles generally have the same magnetic properties as mono-domain particles with higher effective anisotropy. Soft magnetic multi-domain particles with low saturation magnetization have the advantage that they can be shaped into any form for use in the present method according to the invention. If they have an asymmetrical external form, they are then particularly suitable for local viscosity measurements in the area of examination. Soft magnetic multi-domain particles with high saturation magnetization must preferably be designed so that the demagnetizing factor becomes small. Both symmetrical and asymmetrical forms can be considered here. For example, a soft magnetic material with high saturation magnetization can be applied as a thin coating on a ball or cube that is not magnetizable. Soft magnetic multi-domain particles with high saturation magnetization that have an asymmetrical form, e.g. in the form of flakes or needles, can also be used for viscosity measurements.

Therefore, mono-domain particles, where reverse magnetization occurs via Neel and Brownian rotation, are particularly suitable for local viscosity measurements in the area of examination as are soft magnetic multi-domain particles with small or large saturation magnetization that have an asymmetrical external form.

As described above, the magnetic particles also comprise such particles that consist of a non-magnetic core and a coating of a magnetic material. Therefore this comprises in general all magnetic particles that have a low effective anisotropy and those that have a high effective anisotropy. A high coercive force H_{c} is necessary in semi-hard and, especially, hard magnets in order to bring the magnetization to zero. Suitably hard magnetic materials comprise Al-Ni, Al-Ni-Co and Fe-Co-V alloys as well as barium ferrite (BaO 6xFe₂O₃).

In general the magnetic particles in the magnetic particle administering composition, are chosen such that good magnetic particle images, in particular a good resolution can be obtained in a given field gradient. In German patent number 101 51778 a magnetic particle imaging method is described. It is generally described that magnetic mono-domain particles having a size between 20 and 800 nanometres or a glass beat coated with a magnetic coating can be used in this method. However, in order to achieve a good magnetic imaging contrast and resolution at relatively low magnetic field gradients, improved magnetic particle compositions are highly desirable. The inventors have found magnetic particles having improved magnetic particle imaging properties.

Preferably, the magnetic particles in the magnetic particle administering composition have a magnetization curve having a step change, the step change being characterized in that the magnetization change, as measured in an aqueous suspension, in a first field strength window of magnitude delta around the inflection point of said step change is at least a factor 3 higher than the magnetization change in the field strength windows of magnitude delta below or in the field strength windows of magnitude delta above the first field strength window, wherein delta is less than 2000 microtesla, preferably less than 1000 microtesla, and wherein the time in which the magnetisation step change is completed in the first delta window is less than 0.01 seconds, preferably less than 0.005 sec, more preferably less than 0.001, most preferably less than 0.0005 seconds. It has been found, that such magnetic particles are particularly suitable for magnetic particle imaging, in particular for obtaining a good resolution of the image. It is further preferred, that the magnetic particle composition has a magnetisation curve, wherein the step change is at least 10%, preferably at least 20 %, more preferably at least 30 % and most preferably at least 50% of the total magnetisation of the particle composition as measured at an external magnetisation field of 1 Tesla. It is further preferred, that the magnetization change in the first field strength window of magnitude delta around the inflection point of said step change is at least a factor 4, preferably at least a factor 5 higher than the magnetization change in the field strength windows of magnitude delta below or in the field strength windows of magnitude delta above the first field strength window.

The magnetic particle composition is particularly useful for use in a magnetic particle imaging technique. The particles show good spatial resolution at relatively low field strength gradients. Further, the magnetic particle composition allows for a relatively high scanning speed for examining a large examination area. For example, for application in medical magnetic particle imaging, where the step change occurs preferably at a delta value below 1000 microTesla, the particle composition has a resolution value better than between 0.1 and 10 mm at magnetic field strength gradients between 10 and 0.1 T/m. With the magnetic particle imaging technique using the magnetic particle compositions according to the invention extremely good resolution can be obtained, for example in a range from 0.1 to 10 micrometres in applications, where are very high magnetic field is gradients can be achieved, for example in microscopy. It is noted that strictly speaking, magnetic field strength is expressed in H (A/m). However, in the present application, when reference is made to magnetic field strength, B-fields are meant. A magnetic fields B of 2000 µT as described above corresponds to an H field of 2 mT/µ₀ = 1.6 kA/m, that is the equivalent H field that would produce a B field of 2 mT in vacuum.

A method for measuring the magnetisation curve and the required step change is as follows. A sample of a magnetic particle composition is suspended in water, optionally with the help of a simple detergent. To prevent clumping and/or to de-agglomerate the magnetic particles an ultrasound treatment possible can be used. The concentration of the magnetic particle composition is less than O.Olgr core mass per liter of solvent. With core mass is meant the mass of the magnetic material in the magnetic particle composition. The suspension is brought into a fast magnetometer. (i.e. a device that measures the magnetization of the sample while an external field is applied). Suitable fast magnetometers are known to the expert. The magnetometer is equipped with means allowing to produce an external field at the sample position in at least two orthogonal directions simultaneously, i.e. to produce any magnetic field below a given maximum amplitude and a given maximum speed of change. The magnetisation is measured also in at least two orthogonal directions in the same plane.

First the saturation magnetisation is measured. For this, a magnetic field of about one Tesla is applied in one direction and the magnitude of magnetization is measured after at least 10 seconds. Then the measurement sequences for determining the step change starts. The sequence starts with choosing a field vector with an external field magnitude below 20mT. This field is applied for at most 100 seconds. Then a second direction is chosen. This direction defines the scalar values of the field H and the magnetization M. The field is rapidly changed, preferably less than 1 millisecond, so that it lies now in -H direction with some magnitude below 20 mT. Then the field is changed from -H to +H e.g. in a linear way and the (now scalar i.e. projected) magnetization is recorded. The magnetization curve is recorded in less than 0.01s but longer than 1µs. Where the magnetisation curve shows a step change, a first window of size delta is positioned centrally on the inflection point of the magnetisation step change. Similarly, a window of size delta is positioned below and above the first window, and the required step change is evaluated by determining the change in magnetisation in each of the windows.

Whether or not a given magnetic particle composition has the required step change depends in a complicated way on many variables, for example of the size of the particles, the particle size distribution, the shape of the particles, the damping constant for Neel rotation, the type of magnetic material, the crystallinity and the stochiometry of the composition of the magnetic material. It has been found that it is particularly important that the particle size distribution of the particle composition is narrow. Preferably, the magnetic particle composition has a narrow particle size distribution wherein at least 50 weight % of the particles have a particle size between plus or minus 50%, preferably 25%, more preferably 10% of the average particle size. Preferably, the amount of particles within the specified windows, is at least 70 wt %, preferably at least 80 wt %, and most preferably at least 90 wt %. Particularly good results are obtained with mono-domain particles have a low magnetic anisotropy with a field needed for inducing Neel rotation of substantially below 10mT, preferably below 5 mT, more preferably below 2 mT. Preferably, the magnetic particles are mono-domain particles having an average particle size between 20 and 80 nanometres, more preferably between 25 and 70 nanometres, must preferably between 30 and 60 nanometres, wherein at least 50, preferably at least 60, more preferably at least 70 weight % of the particles have a particle size between the average particle size plus or minus 10 nanometre.

The magnetic particle may also be a multi-domain particle having substantially a needle shape having a demagnetisation factor of less than 0.001. This magnetic particle composition is particularly useful in non-medical applications where the needles shape is not a disadvantage. The magnetic particle composition may comprise magnetic particles comprising a non-magnetic core covered with a magnetic coating material, wherein the thickness of the coating is between 5 and 80 nanometres and wherein the demagnetisation factor is less than 0.01 and a diameter below 300µm. Also it is advantageous to have a small particle size distribution as described above. The physical parameters of the magnetic particles are preferably chosen to meet the step change requirement as described above for achieving good imaging properties.

The magnetic particle composition can be manufactured by first forming magnetic particles, for example by precipitation, for example by contacting a solution comprising ferrous and ferric ions with a solution comprising sodium hydroxide as described above. In principle, a known precipitation process can be used. It is also possible to grind the particles from bulk material, for example using a high speed ball mill. The essential next step for obtaining a good magnetic particle composition is the selection and separation of the particles. The first step is to perform a size selection process by filtering and/or centrifuge methods. The next step is to perform a selection process based on the magnetic properties of the particles, for example, using oscillating magnetic gradient fields.

The present invention is based on the surprising recognition that numerous administering forms can be used for the magnetic imaging methods described here using magnetic particles in a gradient magnetic field, whereby the method permits a specific magnetic particle administering form for every analysis task.

The characteristics of the invention described above and in the claims can be used both individually and in any combination for the implementation of the invention in its various embodiments.

## Claims

1. A method to determine the spatial distribution of magnetic particles which have been introduced in an examination area of an object of examination, said method including the following steps:
a) Generation of a magnetic field with a spatial distribution of the magnetic field strength such that the examination area consists of a first sub-area with lower magnetic field strength and a second sub-area with a higher magnetic field strength,
b) Change of the particularly relative spatial position of the two sub-areas in the area of examination or change of the magnetic field strength in the first sub-area so that the magnetization of the magnetic particles which have been introduced in the area of examination in a suspension, aerosol, in the form of a powder, with a casing or, especially, a thin coating, present in at least one capsule, or coupled to cells, particularly white or red blood corpuscles, immune cells, tumor cells or stem cells, or to ingredients, medication, antibodies, transplants or living organisms, or in the form of a, especially liquid, precursor form, changes locally,
c) Acquisition of signals that depend on the magnetization in the area of examination influenced by this change, and
d) Evaluation of signals to obtain information about the change in spatial distribution and/or the movement of the magnetic particles in the area of examination.

2. A method as claimed in claim 1, **characterized in that** the precursor form comprises a first aqueous solution containing FeCl₂ and FeCl₃ and a second aqueous solution containing NaOH, and **in that** the first and second solutions come into contact and form magnetic particles in the area of examination.

3. A method as claimed in claim 1 and 2, **characterized in that** the magnetic particles represent superparamagnetic particles or ferromagnetic particles, particularly in the form of flakes or needles.

4. A method as claimed in any one of the preceding claims, **characterized in that** the area of examination is present in the lungs, sinuses or other parts of the breathing system, in the digestive system, inner ears, bladder, vagina, mammary glands, circulation system, particularly the heart, liver, spleen, lymph system, bone marrow and especially in inflamed organs and/or tumors.

5. A method as claimed in any one of the preceding claims, **characterized in that** the area of examination may comprise boreholes or materials made of plastic or ceramic.

6. A method as claimed in any one of the preceding claims, **characterized in that** steps b) to d) are repeated at least once.

7. A method as claimed in any one of the preceding claims, **characterized in that** the object of examination comprises a polymer material, especially a thermoplastic polymer, or polymer blend, a polymer melt, a micro-organism, a plant, a plant component, an organism or a component of an organism.

8. A method as claimed in any one of the preceding claims, **characterized in that** at least a portion of the magnetic particles has anisotropic properties.

9. A method as claimed in any one of the preceding claims, **characterized in that** the magnetic particle is a mono-domain particle whose magnetic reversal is implemented through Brownian rotation or Neel rotation.

10. A method as claimed in any one of the preceding claims, **characterized in that** the magnetic particle is a hard or soft magnetic multi-domain particle.

11. A method as claimed in any one of the preceding claims, **characterized in that** the magnetic particles comprise hard magnetic materials.

12. A method as claimed in any one of the preceding claims, **characterized in that** the hard magnetic materials comprise Al-Ni, Al-Ni-Co and Fe-Co-V alloys as well as barium ferrite (BaO 6xFe₂O₃).

13. A method as claimed in any one of the preceding claims, **characterized in that** the material used for encasing or coating can be degraded or dissolved thermally, chemically, bio-chemically, by means of electromagnetic radiation or ultrasound and/or mechanically.

## Patentansprüche

1. Verfahren zur Ermittlung der räumlichen Verteilung magnetischer Partikel, die in einen Untersuchungsbereich eines untersuchten Objekts eingeführt wurden, wobei das genannte Verfahren die folgenden Schritte umfasst:
a) Erzeugen eines Magnetfelds mit einer derartigen räumlichen Verteilung der Magnetfeldstärke, dass der Untersuchungsbereich aus einem ersten Teilbereich mit einer geringeren Magnetfeldstärke und einem zweiten Teilbereich mit einer höheren Magnetfeldstärke besteht,
b) Ändern der jeweiligen relativen räumlichen Position der beiden Teilbereiche in dem Untersuchungsbereich oder Ändern der Magnetfeldstärke in dem ersten Teilbereich, so dass sich die Magnetisierung der magnetischen Partikel, die in den Untersuchungsbereich in einer Suspension, einem Aerosol, in der Form eines Pulvers, mit einer Hülle oder insbesondere einer dünnen Beschichtung, vorhanden in mindestens einer Kapsel, oder gekoppelt an Zellen, insbesondere weiße oder rote Blutkörperchen, Immunzellen, Tumorzellen oder Stammzellen, oder an Inhaltsstoffe, Medikamente, Antikörper, Transplantate oder lebende Organismen, oder in der Form einer insbesondere flüssigen Präkursorform eingeführt wurden, lokal verändert,
c) Erfassen von Signalen, die von der durch diese Veränderung beeinflussten Magnetisierung in dem Untersuchungsbereich abhängen, und
d) Evaluieren der Signale, um Informationen über die Änderung in der räumlichen Verteilung und/oder der Bewegung der Magnetpartikel in dem Untersuchungsbereich zu erlangen.

2. Verfahren nach Anspruch 1, **dadurch gekennzeichnet, dass** die Präkursorform eine erste wässrige Lösung mit FeCl₂ und FeCl₃ umfasst und eine zweite wässrige Lösung mit NaOH, und dass die erste und die zweite Lösung in Kontakt kommen und magnetische Partikel in dem Untersuchungsbereich bilden.

3. Verfahren nach den Ansprüchen 1 und 2, **dadurch gekennzeichnet, dass** die magnetischen Partikel superparamagnetische Partikel der ferromagnetische Partikel darstellen, insbesondere in der Form von Flocken oder Nadeln.

4. Verfahren nach einem der vorhergehenden Ansprüche, **dadurch gekennzeichnet, dass** der Untersuchungsbereich in der Lunge, den Nebenhöhlen oder anderen Teilen des Atemtrakts, im Verdauungstrakt, dem Innenohr, der Blase, der Vagina, den Brustdrüsen, dem Kreislaufsystem, insbesondere dem Herzen, der Leber, der Milz, dem Lymphsystem, dem Knochenmark und insbesondere in entzündeten Organen und/oder Tumoren vorhanden ist.

5. Verfahren nach einem der vorhergehenden Ansprüche, **dadurch gekennzeichnet, dass** der Untersuchungsbereich Bohrlöcher oder Material aus Kunststoff oder Keramik umfassen kann.

6. Verfahren nach einem der vorhergehenden Ansprüche, **dadurch gekennzeichnet, dass** die Schritte b) bis d) mindestens ein Mal wiederholt werden.

7. Verfahren nach einem der vorhergehenden Ansprüche, **dadurch gekennzeichnet, dass** das Untersuchungsobjekt ein Polymermaterial, insbesondere ein thermoplastisches Polymer, oder eine Polymermischung, eine Polymerschmelze, einen Mikroorganismus, eine Pflanze, einen Pflanzenbestandteil, einen Organismus oder einen Bestandteil eines Organismus umfasst.

8. Verfahren nach einem der vorhergehenden Ansprüche, **dadurch gekennzeichnet, dass** mindestens ein Teil der magnetischen Partikel anisotrope Eigenschaften hat.

9. Verfahren nach einem der vorhergehenden Ansprüche, **dadurch gekennzeichnet, dass** der magnetische Partikel ein Eindomänen-Partikel ist, dessen magnetische Umkehrung durch Brownsche Rotation oder Neel-Rotation ausgeführt wird.

10. Verfahren nach einem der vorhergehenden Ansprüche, **dadurch gekennzeichnet, dass** der magnetische Partikel ein hart- oder weichmagnetischer Mehrdomainen-Partikel ist.

11. Verfahren nach einem der vorhergehenden Ansprüche, **dadurch gekennzeichnet, dass** die magnetischen Partikel hartmagnetische Materialien umfassen.

12. Verfahren nach einem der vorhergehenden Ansprüche, **dadurch gekennzeichnet, dass** die hartmagnetischen Materialien Al-Ni-, Al-Ni-Co- und Fe-Co-V-Legierungen sowie Bariumferrit (BaO 6xFe₂O₃) umfassen.

13. Verfahren nach einem der vorhergehenden Ansprüche, **dadurch gekennzeichnet, dass** das für die Umhüllung oder Beschichtung verwendete Material thermisch, chemisch, biochemisch, mittels elektromagnetischer Strahlung oder Ultraschall und/oder mechanisch abgebaut oder aufgelöst werden kann.

## Revendications

1. Procédé de détermination de la répartition spatiale de particules magnétiques qui ont été introduites dans une zone d'examen d'un objet d'examen, ledit procédé comprenant les étapes suivantes :
a) la génération d'un champ magnétique avec une répartition spatiale de la force de champ magnétique de sorte que la zone d'examen se compose d'une première sous-zone avec une force de champ magnétique inférieure et d'une deuxième sous-zone avec une force de champ magnétique supérieure,
b) le changement de la position spatiale relative particulière des deux sous-zones dans la zone d'examen ou le changement de la force de champ magnétique dans la première sous-zone afin de changer localement la magnétisation des particules magnétiques qui ont été introduites dans la zone d'examen dans une suspension, un aérosol, sous forme de poudre, avec une enveloppe ou, particulièrement, un revêtement mince, présentes dans au moins une capsule, ou couplées à des cellules, particulièrement des globules blancs ou des globules rouges, des cellules immunes, des cellules de tumeur ou des cellules souches, ou à des ingrédients, des médicaments, des anticorps, des greffes ou des organismes vivants, ou sous forme de précurseur, en particulier liquide,
c) l'acquisition de signaux dépendant de la magnétisation dans la zone d'examen influencée par ce changement, et
d) l'évaluation de signaux pour obtenir des informations sur le changement de répartition spatiale et/ou le déplacement des particules magnétiques dans la zone d'examen.

2. Procédé selon la revendication 1, **caractérisé en ce que** la forme de précurseur comprend une première solution aqueuse contenant FeCl₂ et FeCl₃ et une deuxième solution aqueuse contenant NaOH et **en ce que** les première et deuxième solutions entrent en contact et forment des particules magnétiques dans la zone d'examen.

3. Procédé selon les revendications 1 et 2, **caractérisé en ce que** les particules magnétiques représentent des particules super-paramagnétiques ou des particules ferromagnétiques, particulièrement sous forme de flocons ou d'aiguilles.

4. Procédé selon l'une quelconque des revendications précédentes, **caractérisé en ce que** la zone d'examen est présente dans les poumons, les sinus ou d'autres parties du système respiratoire, dans le système digestif, les oreilles internes, la vessie, le vagin, les glandes mammaires, le système circulatoire, particulièrement le coeur, le foie, la rate, le système lymphatique, la moelle épinière et particulièrement dans des organes enflammés et/ou des tumeurs.

5. Procédé selon l'une quelconque des revendications précédentes, **caractérisé en ce que** la zone d'examen peut comprendre des trous ou des matériaux en plastique ou en céramique.

6. Procédé selon l'une quelconque des revendications précédentes, **caractérisé en ce que** les étapes b) à d) sont répétées au moins une fois.

7. Procédé selon l'une quelconque des revendications précédentes, **caractérisé en ce que** la zone d'examen comprend un matériau polymérique, particulièrement un polymère thermoplastique ou un mélange polymérique, une fusion polymérique, un micro-organisme, une plante, un composant végétal, un organisme ou un composant d'un organisme.

8. Procédé selon l'une quelconque des revendications précédentes, **caractérisé en ce qu'**au moins une portion des particules magnétiques comporte des propriétés anisotropes.

9. Procédé selon l'une quelconque des revendications précédentes, **caractérisé en ce que** la particule magnétique est une particule mono-domaine dont l'inversion magnétique est mise en oeuvre par rotation brownienne ou par rotation de Neel.

10. Procédé selon l'une quelconque des revendications précédentes, **caractérisé en ce que** la particule magnétique est une particule magnétique multi-domaines dure ou souple.

11. Procédé selon l'une quelconque des revendications précédentes, **caractérisé en ce que** les particules magnétiques comprennent des matériaux magnétiques durs.

12. Procédé selon l'une quelconque des revendications précédentes, **caractérisé en ce que** les matériaux magnétiques durs comprennent des alliages Al-Ni, Al-Ni-Co et Fe-Co-V ainsi que de la ferrite de baryum (BaO 6xFe₂O₃).

13. Procédé selon l'une quelconque des revendications précédentes, **caractérisé en ce que** le matériau utilisé pour l'enveloppe ou le revêtement peut être dégradé ou dissout thermiquement, chimiquement, biochimiquement, par rayonnement électromagnétique ou par ultrasons et/ou mécaniquement.
